Europäisches Patentamt

European Patent Office

Office européen des brevets

(19)

(11) **EP 0 984 784 B1**

(12) **FASCICULE DE BREVET EUROPEEN**

(45) Date de publication et mention
de la délivrance du brevet:
**20.11.2002 Bulletin 2002/47**

(51) Int Cl.$^7$: **A61K 31/675**, A61K 31/70,
A61K 31/165, A61K 31/475,
C07K 14/47, A61P 35/00

(21) Numéro de dépôt: **98928381.7**

(22) Date de dépôt: **28.05.1998**

(86) Numéro de dépôt international:
**PCT/FR98/01074**

(87) Numéro de publication internationale:
**WO 98/053839 (03.12.1998 Gazette 1998/48)**

(54) **PRODUITS ANTI-CANCEREUX POUR LE TRAITEMENT DE LA MUCOVISCIDOSE**

ANTITUMORALE VERBINDUNGEN ZUR BEHANDLUNG VON MUCOVISCIDOSIS

ANTI-CANCER PRODUCTS FOR TREATING CYSTIC FIBROSIS

(84) Etats contractants désignés:
**AT BE CH CY DE DK ES FI FR GB GR IE IT LI LU
MC NL PT SE**

(30) Priorité: **30.05.1997 FR 9706667**

(43) Date de publication de la demande:
**15.03.2000 Bulletin 2000/11**

(73) Titulaire: **CENTRE NATIONAL DE
LA RECHERCHE SCIENTIFIQUE
75016 Paris Cédex (FR)**

(72) Inventeurs:
• **STOVEN, Véronique**
  **F-75006 Paris (FR)**
• **LENOIR, Gérard**
  **F-75007 Paris (FR)**
• **LALLEMAND, Jean-Yves**
  **F-91120 Palaiseau (FR)**
• **ANNEREAU, Jean-Philippe**
  **F-75015 Paris (FR)**
• **BARTHE, Joel**
  **F-75007 Paris (FR)**
• **BLANQUET, Sylvain**
  **F-75013 Paris (FR)**

(74) Mandataire: **Warcoin, Jacques et al
Cabinet Régimbeau
20, rue de Chazelles
75847 Paris cedex 17 (FR)**

(56) Documents cités:
**EP-A- 0 715 853        WO-A-94/25607
WO-A-96/01627**

• **JEDLITSCHKY G ET AL: "TRANSPORT OF
GLUTATHIONE, GLUCURONATE, AND
SULFATE CONJUGATES BY THE MRP
GENE-ENCODED CONJUGATE EXPORT PUMP"
CANCER RESEARCH, vol. 56, 1 mars 1996,
pages 988-994, XP002004824**
• **AKIMARU: "IINDUCTION OF MRP/GS-X PUMP
AND CELLULAR RESISTANCE TO
ANTICANCER PROSTAGLANDINS"
CYTOTECHNOLOGY, vol. 19, no. 3, 1996, pages
221-227, XP002055208**
• **YI J -R ET AL: "EXPRESSION CLONING OF THE
CDNA FOR A POLYPEPTIDE ASSOCIATED
WITH RAT HEPATIC SINUSOIDAL REDUCED
GLUTATHIONE TRANSPORT:
CHARACTERISTICS AND COMPARISON WITH
THE CANALICULAR TRANSPORTER"
PROCEEDINGS OF THE NATIONAL ACADEMY
OF SCIENCES OF USA, vol. 92, no. 5, février
1995, pages 1495-1499, XP002017516**
• **JEDLINSCHKY: "ATP-DEPENDENT
TRANSPORT OF GLUTATHIONE
S-CONJUGATES BY THE MULTIDRUG
RESISTANCE-ASSOCIATED PROTEIN"
CANCER RES., vol. 54, no. 18, 1994, pages
4833-4836, XP002055209**
• **JEDLITSCHKY G ET AL: "ATP-DEPENDENT
GLUTATHLONE CONJUGATE TRANSPORT IN
HL60 CELLS OVEREXPRESSING THE
MULTIDRUG RESISTANCE ASSOCIATED
PROTEIN (MRP)" ANTI-CANCER DRUGS, vol. 5,
no. SUPPL. 01, 1 septembre 1994, page 4
XP000570353**

- MUELLER M ET AL: "OVEREXPRESSION OF THE GENE ENCODING THE MULTIDRUG RESISTANCE-ASOCIATED PROTEIN RESULTS IN INCREASED ATP-DEPENDENT GLUTATHIONE S-CONJUGATE TRANSPORT" PROCEEDINGS OF THE NATIONAL ACADEMY OF SCIENCES OF USA, vol. 91, 1 décembre 1994, pages 13033-13037, XP002004825
- ISHIKAWA: "HOW DOES THE MRP/GS-X PUMP EXPORT DOXORUBICIN ?" J. NATL CANCER INST., vol. 87, no. 21, 1995, pages 1639-1640, XP002055210
- LEIER I ET AL: "ATP-DEPENDENT GLUTATHIONE DISULPHIDE TRANSPORT MEDIATED BY THE MRP GENE-ENCODED CONJUGATE EXPORT PUMP" BIOCHEMICAL JOURNAL, vol. 314, no. 2, 1 mars 1996, pages 433-437, XP000570352
- DEMOLOMBE S ET AL: "ATP-binding cassette proteins as targets for drug discovery" TRENDS IN PHARMACOLOGICAL SCIENCES, vol. 17, no. 8, août 1996, page 273-275 XP004034573
- ISHIKAWA: "THE GS-X PUMP IN PLANT, YEAST, AND ANIMAL CELLS: FUNCTION AND GENE EXPRESSION" BIOSCI. REPORTS, vol. 17, no. 2, avril 1997, pages 189-207, XP002055211
- KO: "THE CYSTIC FIBROSIS TRANSMEMBRANE CONDUCTANCE REGULATOR" J. BIOL. CHEM., vol. 268, no. 32, 1993, pages 24330-24338, XP002055212
- KO: "THE FIRST NUCLEOTIDE BINDING FOLD OF THE CYSTIC FIBROSIS CONDUCTANCE REGULATOR CAN FUNCTION AS AN ACTIVE ATPASE" J. BIOL. CHEM., vol. 270, no. 38, 22 septembre 1995, pages 22093-22096, XP002055213
- COHN: "REGULATION OF THE NBD1/R DOMAIN OF CFTR BY ATP AND BY PROTEIN KINASES" GASTROENTEROLOGY, vol. 112, no. 4SUPPL., avril 1997, page A355 XP002055214
- MAYER R ET AL: "EXPRESSION OF THE MRP GENE-ENCODED CONJUGATE EXPORT PUMP IN LIVER AND ITS SELECTIVE ABSENCE FROM THE CANALICULAR MEMBRANE IN TRANSPORT-DEFICIENT MUTANT HEPATOCYTES" THE JOURNAL OF CELL BIOLOGY, vol. 131, no. 1, octobre 1995, pages 137-150, XP000608636
- GUAY-BRODER C ET AL: "A1 RECEPTOR ANTAGONIST 8-CYCLOPENTYL-1,3-DIPROPYLXANTHINE SELECTIVELY ACTIVATES CHLORIDE EFFLUX FROM HUMAN EPITHELIAL AND MOUSE FIBROBLAST CELL LINES EXPRESSING THE CYSTIC FIBROSIS TRANSMEMBRANE REGULATOR DELTAF508 MUTATION" BIOCHEMISTRY, vol. 34, no. 28, 18 juillet 1995, pages 9079-9087, XP000565690
- CHEN S H ET AL: "FUNCTIONAL ACTIVATION OF THE CYSTIC FIBROSIS TRAFFICKING MUTANT F508-CFTR BY OVEREXPRESSION" AMERICAN JOURNAL OF PHYSIOLOGY. LUNG CELLULAR AND MOLECULAR PHYSIOLOGY, vol. 268, no. 4, PART 01, 1995, pages L615-L624, XP002064541
- BREUER W ET AL: "Induction of multidrug resistance downregulates the expression of CFTR in colon epithelial cells." AMERICAN JOURNAL OF PHYSIOLOGY, (1993 DEC) 265 (6 PT 1) C1711-5. JOURNAL CODE: 3U8. ISSN: 0002-9513., XP002084199 United States
- KOBAYASHI H.: "Biofilm Disease: Its Clinical MAnifestation and THerapeutic possibilities of Macrolides" AM. J. MEDICINE, vol. 99, no. 6 part A, 1995, pages 26S-30S, XP002084200
- REINERT P.: "Effects de l'azithromycine sur la virulence du pyocyanique" PATHOLOGIE BIOLOGIE, vol. 43, no. 6, 1995, pages 551-553, XP002084201
- PUCHELLE E: "CFTR (CYSTIC FIBROSIS TRANSMEMBRANE CONDUCTANCE REGULATOR): UNE PROTEINE A MULTIPLES FONCTIONS" M/S MEDECINE SCIENCES, vol. 10, no. 6/07, juin 1994, pages 627-629, XP000648271
- DAGLI ET AL.: "Use of low-dose Methotrexate for anti-inflammatory treatment of advanced cystic fibrosis" AM. REV. RESPIR. DIS., vol. 141, no. 4 part 2, 1990, page A812 XP002084202
- BAUERNFEIND, A. ET AL: "Staphylococcal aspects of cystic fibrosis. Selection of antibiotics for treatment and prophylaxis of staphylococcal infections in cystic fibrosis patients" INFECTION (MUNICH) (1990), 18(2), 126-30 CODEN: IFTNAL;ISSN: 0300-8126, XP002084203
- LALLEMAND J.Y. ET AL: "Induction by antitumoral drugs of proteins that functionally complement CFTR: A novel therapy for cystic fibrosis ? [2]." LANCET, (1997) 350/9079 (711-712). REFS: 5 ISSN: 0140-6736 CODEN: LANCAO, XP002084204 United Kingdom
- KUWERTZ-BROKING E ET AL: "Colchicine for secondary nephropathic amyloidosis in cystic fibrosis [letter]." LANCET, (1995 MAY 6) 345 (8958) 1178-9. JOURNAL CODE: L0S. ISSN: 0140-6736., XP002084205 ENGLAND: United Kingdom
- MACLEOD R J ET AL: "Developmental differences of cystic fibrosis transmembrane conductance regulator functional expression in isolated rat fetal distal airway epithelial cells." PEDIATRIC RESEARCH, (1994 JAN) 35 (1) 45-9. JOURNAL CODE: OWL. ISSN: 0031-3998., XP002084206 United States
- ROUM J H ET AL: "SYSTEMIC DEFICIENCY OF GLUTATHIONE IN CYSTIC FIBROSIS" JOURNAL OF APPLIED PHYSIOLOGY, vol. 75, no. 6, décembre 1993, pages 2419-2424, XP002061635

- **MAAYAN CH ET AL: "IMMEDIATE EFFECT OF VARIOUS TREATMENTS ON LUNG FUNCTION IN INFANTS WITH CYSTIC FIBROSIS" RESPIRATION, vol. 55, no. 3, août 1989, pages 144-151, XP000603262**
- **FISCHER ET AL.: "The actin filament disrupter cytochalasin D activates the recombinant cystic fibrosis transmembrane conductance regulator Cl-channel in mouse 3T3 fibroblasts" JOURNAL OF PHYSIOLOGY, vol. 489, no. part 3, 1995, pages 745-754, XP002084224**
- **ANNEREAU ET AL.: "Insight into cystic fibrosis by structural modeling of CFTR first nucleotide binding fold (NBF1)." C.R.ACAD. SCI., SER III, vol. 320, no. 2, 1997, pages 113-121, XP002085670**
- **ANNEREAU J -P ET AL: "A NOVEL MODEL FOR THE FIRST NUCLEOTIDE BINDING DOMAIN OF THE CYSTIC FIBROSIS TRANSMEMBRANE CONDUCTANCE REGULATOR" FEBS LETTERS, vol. 407, no. 3, 5 mai 1997, pages 303-308, XP002061632 cité dans la demande**

LISTE DE SEQUENCES

(1) INFORMATIONS GENERALES:

(i) DEPOSANT:

(A) NOM: CENTRE NATIONALE DE LA RECHERCHE SCIENTIFIQUE (CNRS)
(B) RUE: 3, RUE MICHEL ANGE
(C) VILLE: PARIS Cedex 16
(E) PAYS: FRANCE
(F) CODE POSTAL: 75794

(ii) TITRE DE L' INVENTION: PRODUITS ANTI-CANCEREUX POUR LE TRAITEMENT DE LA MUCOVISCIDOSE

(iii) NOMBRE DE SEQUENCES: 2

(iv) FORME DECHIFFRABLE PAR ORDINATEUR:

(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(vi) DONNEES DE LA DEMANDE ANTERIEURE:

(A) NUMERO DE LA DEMANDE: FR 9706667

(B) DATE DE DEPOT: 30-MAY-1997

(2) INFORMATIONS POUR LA SEQ ID NO: 1:

(i) CARACTERISTIQUES DE LA SEQUENCE:

(A) LONGUEUR: 22 paires de bases
(B) TYPE: nucléotide
(C) NOMBRE DE BRINS: simple
(D) CONFIGURATION: linéaire

(ii) TYPE DE MOLECULE: ADN

(ix) CARACTERISTIQUE:

(A) NOM/CLE: oligonucléotide 5'-3'

(xi) DESCRIPTION DE LA SEQUENCE: SEQ ID NO: 1:

```
GCAGATCTAG AGGACAGTTG TT                                    22
```

(2) INFORMATIONS POUR LA SEQ ID NO: 2:

(i) CARACTERISTIQUES DE LA SEQUENCE:

(A) LONGUEUR: 31 paires de bases
(B) TYPE: nucléotide
(C) NOMBRE DE BRINS: simple
(D) CONFIGURATION: linéaire

(ii) TYPE DE MOLECULE: ADN
(ix) CARACTERISTIQUE:

(A) NOM/CLE: oligonucléotide 3'-5'

(xi) DESCRIPTION DE LA SEQUENCE: SEQ ID NO: 2:

```
TACAAAATTG TCTTTTTCTT ATTCTTAAGC G                          31
```

## Description

La présente invention concerne une nouvelle approche pour le traitement de la mucoviscidose, qui fait intervenir la chimiothérapie, notamment anti-cancéreuse.

La mucoviscidose est une maladie génétique d'expression surtout pulmonaire qui relève d'un défaut dans le gène codant pour la protéine CFTR (pour « Cystic Fibrosis Transmembrane Conductance Regulator »), protéine capable de participer directement ou indirectement au transport des ions chlorures à travers les membranes cellulaires.

D'une manière générale, la protéine CFTR appartient à la famille des transporteurs ABC (pour « ATP Binding Cassette »), une famille très large de protéines dont les membres se retrouvent tant chez les eucaryotes que chez les procaryotes. Ce sont en général des transporteurs actifs qui hydrolysent l'ATP pour fournir le potentiel chimique nécessaire à leur fonction de transport. Ainsi, chez les eucaryotes, elles transportent différents types de molécules à travers les membranes cellulaires. Parmi les molécules susceptibles d'être transportées, on peut citer les ions, les vitamines, les peptides, les sucres ainsi que les substances médicamenteuses ou autres drogues. Leur organisation globale présente des caractères communs : elles comprennent généralement une région transmembranaire (TM) qui participe à la sélection de l'entité chimique à transporter, et un domaine de fixation des nucléotides qui hydrolyse l'ATP pour fournir le potentiel chimique nécessaire au transport (NBF pour « Nucleotide Binding Fold »).

Les gènes codant pour ces protéines sont souvent issus de la fusion de deux gènes de structure analogue.

La structure des protéines correspondantes est, alors généralement la suivante :

$$(TM1)-(NBF1)-(TM2)-(NBF2)$$

La protéine CFTR représente un élément de 1480 acides aminés d'une sous-famille de la famille des transporteurs ABC appelée sous-famille MRP/CFTR. Outre la protéine CFTR, cette sous-famille comprend la protéine MRP humaine. Les deux protéines présentent en effet une similarité de séquence d'environ 50 %. La protéine MRP (pour « Multi-drug Resistance associated Protein »), est connue pour être impliquée dans des phénomènes de multi-résistance aux médicaments utilisés en chimiothérapie du cancer (M. Dean, R. Allikmets, Current Opinion in Genetics & Development, 5, 779-785, 1995). La protéine CFTR est également très proche structurellement d'un autre membre de la sous-famille MRP/CFTR, la protéine YCF1 (pour « Yeast Cadmium Resistance Factor 1 ») qui confère à la levure Saccharomyces cerevisiae le phénotype de résistance aux ions cadmium (Tommasini et al., PNAS, 93, 6743-6748, 1996). Outre leur similarité de structure, les protéines MRP et YCF1 présentent un mode de fonctionnement analogue : elles exportent les molécules et les ions sous la forme de leurs adduits avec le glutathion (G.J. Zaman et al., PNAS, 92, 7690-7694, 1995).

La protéine CFTR présente également des analogies de structure non négligeables avec la protéine STE6 de levure, qui transporte la phéromone « facteur a » chez la levure Saccharomyces cerevisiae (J.L. Teem et al., Cell, 73, 335-346, 1993) ainsi qu'avec la protéine MDR humaine (pour « Multi-drug Resistance Protein ») connue comme MRP, pour être impliquée dans les phénomènes de multi-résistance aux médicaments anti-cancéreux. La protéine MDR humaine et la protéine STE6 appartiennent à une autre sous-famille des transporteurs ABC, appelée MDR/TAP.

Ainsi l'appartenance générale de la protéine CFTR à la famille des transporteurs ABC ainsi que sa fonction de transporteur des ions chlorure sont maintenant bien connues.

Chez un malade atteint de la mucoviscidose, la protéine CFTR est mutée. Plus de 600 mutations ont été inventoriées mais dans environ 70 % des cas, la mutation en cause est une délétion d'une phénylalanine en position 508, dans la partie NBF1 (ΔF508) de la structure globale de la protéine. Il semble que cette mutation entraîne un défaut de repliement de la protéine qui est alors détruite à l'intérieur de la cellule sans achever sa maturation post-traductionnelle (Riordan J.R., Rommens J.M., Kerem B.S., Alon N., Rozmahel R., Grzelczack Z., Zielenski J., Lok S., Plavic N., Chou J.L., Drumm M.L., Iannuzzi M.C., Collins F.S., Tsui L.C. (1989) Science. 245, 1066-1072). L'absence d'une protéine CFTR mature ou fonctionnelle conduit à un défaut de sécrétion des ions chlorures. Le test dit « de la sueur », qui mesure la sécrétion des ions chlorures, a d'ailleurs été mis au point dès 1953, et reste indispensable pour diagnostiquer la mucoviscidose.

Jusqu'à présent des tentatives ont été faites pour traiter la mucoviscidose par la thérapie génique, en développant des systèmes d'administration aux malades d'un acide nucléique codant pour la protéine CFTR sauvage, vectorisé soit par des virus, soit par des lipides cationiques. Des essais d'administration d'un tel complexe ADN/lipides cationiques ont été faits à des poumons de souris par voie intra-trachéale (Yoshimura et al. « Nucleic Acid Research, 20 :3233-3240, 1992) ou encore par aérosol (Stribling et al., Proc. Natl. Acad. Sci. 89 : 11277-11281, 1992). On a également constaté que l'administration du gène codant pour la CFTR complexé avec des lipides cationiques à une souris modèle souffrant de mucoviscidose, avait pour effet la correction du défaut de la fonction de canal à ion chlorure (Hyde et al., Nature 362 : 250-255, 1993).

Ainsi, les approches thérapeutiques envisagées à l'heure actuelle pour la mucoviscidose, visent uniquement le défaut génétique (mutation dans le gène codant pour la protéine CFTR) qu'elles s'efforcent de corriger. Elles lient l'efficacité du traitement au rétablissement de la seule fonction de canal à ion chlorure exercée par une protéine CFTR fonctionnelle.

Cependant, bien que le défaut de transport des ions chlorure constitue effectivement une manifestation clinique de la mucoviscidose, d'autres manifestations ne sont pas encore totalement expliquées.

Ainsi par exemple, les organes principalement atteints dans la mucoviscidose sont ceux dans lesquels le glutathion est sécrèté, notamment le foie, ou ceux dans lesquels des mécanismes de détoxification sont susceptibles de faire intervenir le glutathion (poumons, intestin, colon).

Par ailleurs, on a constaté que chez les patients atteints de mucoviscidose, la réaction inflammatoire est excessive. On constate souvent chez ces patients une inflammation chronique des voies respiratoires. Dans ce cas, un nombre très élevé de polynucléaires neutrophiles est présent dans les voies respiratoires des malades, y compris en l'absence d'infection détectable. Il est possible que cette inflammation précède l'apparition de l'infection chronique. Cet afflux de polynucléaires neutrophiles engendre la libération massive de radicaux libres et d'hyper oxydes dans les cellules des voies respiratoires des malades. Or, on sait que le glutathion extra et intra cellulaire joue un rôle central dans le contrôle de la réaction inflammatoire en protégeant les cellules de l'agression de ces oxydants. Or cette protection semble altérée chez les malades atteints de mucoviscidose. Il semblerait donc que la mucoviscidose empêche le glutathion de jouer son rôle habituel.

Des études menées sur la famille des protéines transporteur ABC ont mis en évidence leur relative versatilité. Bien que possédant chacune une fonction propre, elles semblent présenter un mode de fonctionnement commun qui leur permet d'assurer des fonctions communes. Par exemple, il a été démontré in vitro que la protéine MRP humaine pouvait se substituer à YCF1 chez la levure et y assurer la fonction de détoxification du cadmium (Tommasini et al., PNAS, 93, 6743-6748, 1996). Elle peut également se substituer à la protéine STE6 chez la levure pour y assurer le transport du facteur a (J.L. Teem et al., Cell, 73, 335-346, 1993). De même, une protéine chimère de STE6, dans laquelle le domaine NBF1 de CFTR a été substitué au domaine NBF1 de STE6 est fonctionnelle et transporte effectivement le facteur a (J.L. Teem et al., Cell, 73, 335-346, 1993). Enfin, in vivo il semblerait que les gènes codant pour les protéines CFTR, MDR et MRP soient sous des contrôles transcriptionnels couplés (Trezise A.E., Romano P.R., Gill D.R., Hyde S.C., Sepulveda F.V., Buchwald M., Higgins C.F., EMBO J., 11, 4291-4303, 1992 & M.A. Izquierdo, J. J. Neezfjes, A.E.L. Mathari, M.J. Flens, G.L. Scheffer, R.J. Scheper, British Journal of Cancer, 74, 1961-1967, 1996).

Les inventeurs ont maintenant trouvé que de façon surprenante, cette versatilité des protéines transporteur ABC pouvait être utilisée pour envisager un traitement de la mucoviscidose par chimiothérapie, en administrant au patient certains types de produits. Ladite administration conduirait à l'expression ou à la surexpression d'un composé qui peut jouer le rôle d'une protéine CFTR fonctionnelle et donc pallier les déficiences de la protéine CFTR mutée.

C'est pourquoi l'invention a pour objet l'utilisation, pour la préparation d'un médicament destiné à la prévention et/ou au traitement de la mucovisidose, d'au moins un produit dont l'administration à un patient atteint de mucovisidose, conduit chez ledit patient à l'expression ou à la surexpression d'au moins un composé transporteur ABC.

On suppose que pour le traitement de la mucoviscidose, le mécanisme d'action du composé exprimé ou surexprimé est tel qu'il peut se substituer à la protéine CFTR déficiente chez le malade. On suppose que le composé exerce alors une fonction normalement remplie par la protéine CFTR sauvage et que la protéine CFTR déficiente ne sait pas remplir chez le patient atteint de mucoviscidose.

Ainsi, des produits utilisables pour le traitement de la mucoviscidose sont ceux qui peuvent induire dans l'organisme du patient l'expression ou la surexpression d'un composé transporteur ABC, lequel se substitue à la protéine CFTR déficiente.

Différents modes de réalisation sont envisageables. Selon un premier mode de réalisation, on peut envisager d'administrer des produits qui induisent l'expression ou la surexpression de la protéine CFTR mutée du malade. Elle serait alors exprimée à un niveau tel que, malgré la présence d'une mutation, elle retrouverait sa fonctionnalité.

Selon un autre mode de réalisation, on sélectionnera des produits conduisant à l'expression ou à la surexpression de la protéine MDR.

Par ailleurs, les travaux menés par les inventeurs suggèrent que la protéine CFTR joue en plus de son rôle de canal à ions chlorures, un rôle de pompe à glutathion. Cette hypothèse pourrait rendre compte de certaines manifestations cliniques présentées par les malades atteints de mucoviscidose, chez qui la protéine CFTR n'est pas fonctionnelle.

De plus, les inventeurs ont mis en évidence, dans le domaine NBF1 de la protéine CFTR, l'existence d'un site potentiel de fixation du glutathion, ce qui peut rendre compte du rôle joué par le glutathion dans la fonction de transporteur exercée par la protéine CFTR. Le rôle de pompe à glutathion de la protéine CFTR permettrait notamment de mieux comprendre l'inflammation chronique caractéristique de la mucoviscidose. En effet, le glutathion est une molécule clé dans le déclenchement et le contrôle de la réaction inflammatoire. Il intervient dans le métabolisme de composés pro-inflammatoires tels que les leukotriènes mais également dans le contrôle de ces réactions en tant qu'agent protecteur vis à vis des oxydants libérés par les polynucléaires neutrophiles (radicaux, hydroperoxydes...).

C'est pourquoi, l'invention concerne plus particulièrement l'utilisation, pour la préparation d'un médicament destiné à la prévention et/ou au traitement de la mucoviscidose d'au moins un produit dont l'administration à un patient atteint de mucoviscidose, conduit chez ledit patient à l'expression ou à la surexpression d'un composé ABC transporteur de glutathion.

Par « composé transporteur de glutathion », on entend selon l'invention, un composé qui appartient à la famille déjà répertoriée des transporteurs ABC qui exercent leur fonction de transport par l'intermédiaire du glutathion. On entend également tout fragment ou analogue d'un tel transporteur résultant d'une ou plusieurs mutations, qui conserve la propriété de transport par l'intermédiaire du glutathion.

On entend par glutathion, le glutathion lui-même ou ses adduits avec d'autres composés. Il peut s'agir d'adduits naturels, tels que les leukotriènes, ou encore d'adduits de détoxification, avec les métaux lourds, les oxydants puissants (radicaux libres, peroxydes...), ou les drogues.

Il est en effet, déjà établi qu'un certain nombre de protéines, notamment la protéine MRP humaine, exportent les drogues et médicaments, notamment les produits anti-cancéreux, par l'intermédiaire du glutathion, soit sous forme d'adduits directs glutathion-drogue, soit par fixation simultanée ou séquentielle du glutathion et de la drogue (G.J. Zaman et al., PNAS, 92, 7690-7694, 1995).

Selon un mode particulièrement préféré, on cherchera à exprimer ou à surexprimer la protéine MRP. Dans ce cas, mais également dans le cas plus général où le composé exprimé ou surexprimé est un transporteur ABC, notamment la protéine MDR, les produits utilisables selon l'invention sont de préférence des produits anti-cancéreux de type antinéoplasiques, c'est-à-dire des agents anti-cancéreux cytotoxiques qui, pour le traitement du cancer, doivent tuer aussi sélectivement que possible, les cellules cancéreuses cibles. En effet, il est connu que l'administration de ces agents anti-cancéreux chez des patients atteints de cancer a souvent tendance à générer des phénomènes de résistance aux anti- cancéreux de type pharmacocinétique et causés par la surexpression de protéines de type MRP ou MDR (M. Dean, R. Allikmets, Current Opinion in Genetics & Development, 5, 779-785, 1995 ; Jedlitschky et al., Cancer Research, vol. 56, 1er mars 1996, 988-994 ; Akimaru, Cytotechnology, vol. 19, n° 3, 196, 221-227 ; Jedlitschky, Cancer Research., vol. 54, n° 18, 1994, 4833-4836 ; Jedlitschky et al., Anti-cancer drugs, vol. 5 suppl. 01, septembre 1994, 4 ; Mueller et al., PNAS, vol. 91, 1994, 13033-13037 ; Ishikawa, J. Natl. Cancer Inst., vol 87, n° 21, 1995, 1639-1640 ; Leier et al. Biochemical Journal, vol. 314, n° 2, 1996, 433-437).

L'invention vise donc l'utilisation pour le traitement de la mucoviscidose des quatre grandes familles d'antinéoplasiques utilisées dans le traitement du cancer : les agents alkylants, les agents intercalants, les antimétabolites et les poisons du fuseau.

Les agents alkylants sont des agents capables de remplacer un proton d'une molécule par un groupe alkyle. Ils modifient la structure de l'ADN de façon significative au moment des mitoses dont ils perturbent le déroulement. Parmi les grandes familles d'agents alkylants utilisables selon l'invention, on peut citer les moutardes azotées, les nitroso-urées, les dérivés du platine, les dérivés de l'éthylène-imine, les diméthane sulfonoxy-alkanes, les dérivés de pipérazine, les dérivés de la méthylhydrazine. Des exemples représentatifs des moutardes azotées sont le chlorambucil, le cyclophosphamide, l'ifosfamide, l'estramustine, le melphalan, le chlorméthine. Avantageusement, on utilise l'ifosfamide, notamment en combinaison avec un agent intercalant.

Les agents intercalants, sont des agents qui s'intercalent entre les deux bras complémentaires de l'ADN, bloquant ainsi la réplication de l'ADN, la transcription en ARNm et la synthèse protéique. Les grandes familles utilisables selon l'invention comprennent, de façon non limitative, les anthracyclines, les anthracérédiones, les anthracènes, les dérivés de l'acridine, les ellipticines et les actinomycines. De façon préférée, on utilise les anthracyclines, parmi lesquelles on peut citer de façon non limitative la clarubicine, la doxorubicine, la daunorubicine, l'épirubicine, l'idarubicine, la zorubicine et la pirabucine. De façon préférée, on utilisera l'épirubicine, notamment en combinaison avec l'ifosfamide.

Une troisième famille de composés utilisables selon l'invention est représentée par les antimétabolites, ou antogonistes, ou analogues structuraux qui inhibent habituellement une ou plusieurs étapes de la synthèse des acides nucléiques. Ses composés sont représentés de façon non limitative par les antifoliques, les antipuriques et les antipyrimidiques. On peut citer en particulier, l'améthoptérine (méthotrexate), la mercaptopurine, le 5 fluoro-uracile ou encore la cytarabine.

Une autre catégorie d'agents anti-cancéreux est représentée par les poisons du fuseau qui bloquent la mitose cellulaire. Ce sont les anti-mitotiques dont les familles représentatives sont les épipodophyllotoxines et les vinca-alcaloïdes. On peut citer parmi les épipodophylotoxines le téniposide ou encore l'étoposide. Parmi les dérivés de vinca, on peut citer par exemple, la vindésine, la vinorébine, la vincristine ou encore la vinblastine. On citera de préférence la colchicine et ses dérivés. Des résultats particulièrement satisfaisants ont été obtenus avec la colchicine, ce qui est d'autant plus avantageux que ce produit est connu pour être peu toxique.

Enfin, il faut également citer la famille des taxoïdes (taxol, taxotère...).

Selon un autre mode de réalisation, on peut également utiliser des agents cytolytiques divers tels que par exemple la bléomycine, la dacarbazine, l'hydroxycarbamide l'asparaginase, la mitoguazone ou encore la plicamycine.

On citera plus particulièrement le phénylbutyrate de sodium, un agent cytostatique utilisé dans les maladies du cycle de l'urée avec manifestations hépatiques.

On citera également à titre de produits utilisables selon l'invention, les produits de la famille des macrolides qui sont connus pour leurs propriétés d'induction de la surexpression de la protéine MDR conduisant au phénomène de résistance aux médicaments (MDR). Des exemples de ces inducteurs sont ceux cités dans la publication Seelig Eur. J. Biochem., 25, 252-261 (1998).

Certains ont déjà été cités comme faisant partie des agents anti-cancéreux. Ces inducteurs comprennent en particulier, l'actinomycine D, le clotrimazole, la colchicine, la daunorubicine, la doxorubicine, l'epothilone A, l'erythromycine, l'eroposide, l'isosafrole, le midazolame, la nifedipine, le phenobarbital, la puromycine, la reserpine, la rifampicine, le taxol, la vinblastine, la vincristine, la cysteine methylester, l'epinephrine le farnosol.

A titre d'exemple, on citera préférentiellement l'azithromycine, administrée à des doses inférieures à celles nécessaires pour obtenir une activité anti-bactérienne (Jaffé et al., Lancet 1998 ; 351:420).

Un autre exemple de macrolide inducteur de la protéine MDR utilisable selon l'invention est l'erythromycine (Grant et al;, Toxicol. Appl. Pharmacol., 1995 ; 133:269-76)

D'une façon plus générale, les travaux menés par les inventeurs permettent de procéder à une sélection de produits susceptibles d'être utiles dans la prévention et/ou le traitement de la mucoviscidose, en dosant les ARNm des protéines CFTR, MRP et MDR dans les cellules des patients. Selon un mode de réalisation préféré, les produits utiles sont donc ceux dont l'administration conduit à l'apparition ou à une augmentation des ARNm correspondant à ces protéines, et donc en particulier, à une expression ou surexpression de la protéine MRP et/ou MDR et/ou éventuellement CFTR elle-même.

Il peut être particulièrement intéressant de tester par exemple des produits qui ne sont pas suffisamment toxiques pour les cellules, pour être actifs en thérapie anti-cancéreuse, mais cependant suffisamment actifs pour générer un phénomène de "multi drug resistance" (MDR), mis à profit dans le cadre de la présente invention.

Tous ces composés peuvent être utilisés dans des conditions qui suffisent à déclencher l'expression ou la surexpression de la protéine MRP et/ou MDR et/ou CFTR.

La polychimiothérapie par voie générale, intermittente et séquentielle est couramment utilisée pour la thérapie du cancer. On peut également envisager d'utiliser ce type de traitement selon l'invention.

L'invention a donc également pour objet l'utilisation pour la préparation d'un médicament destiné à la prévention et/ou au traitement de la mucoviscidose d'un produit contenant au moins un agent anti-cancéreux et/ou un macrolide, comme produit de combinaison pour une utilisation simultanée, séparée ou étalée dans le temps.

De préférence, ledit produit de combinaison comprendra un agent alkylant et un agent intercalant et de façon encore préférée l'agent alkylant est l'ifosfamide et l'agent intercalant est l'épirubicine.

Les produits utilisables selon l'invention sont de façon préférée administrés avec un précurseur de glutathion, pour une utilisation simultanée, séparée ou étalée dans le temps. On a en effet souvent constaté que les malades atteints de mucoviscidose sont déficients en glutathion et l'administration d'un précurseur de glutathion permet de favoriser une augmentation du taux de glutathion. Lorsque les produits administrés sont des produits anti-cancéreux qui induisent l'expression ou la surexpression de la protéine MRP, l'administration conjointe d'un précurseur de glutathion est particulièrement souhaitable, puisque l'activité de la protéine MRP dépend du glutathion. Parmi les précurseurs de glutathion, on peut citer par exemple le N-acétyle cystéine (par exemple celle commercialisée sous l'appellation Mucomyst) ou encore la N-acétyle lysine.

Par ailleurs on peut administrer au patient un composé qui remplace directement la protéine CFTR déficiente en jouant le rôle de transporteur de glutathion.

On peut ainsi utiliser un composé transporteur de glutathion, à titre de médicament pour la prévention secondaire et/ou le traitement de la mucoviscidose. Le clonage d'un composé transporteur de glutathion dans le foie du rat a déjà été réalisé par certains auteurs (Yi et al., PNAS vol. 92, n° 5, 1995, 1495-1499). Cependant l'éventualité d'un lien avec la mucoviscidose n'a jamais été envisagée.

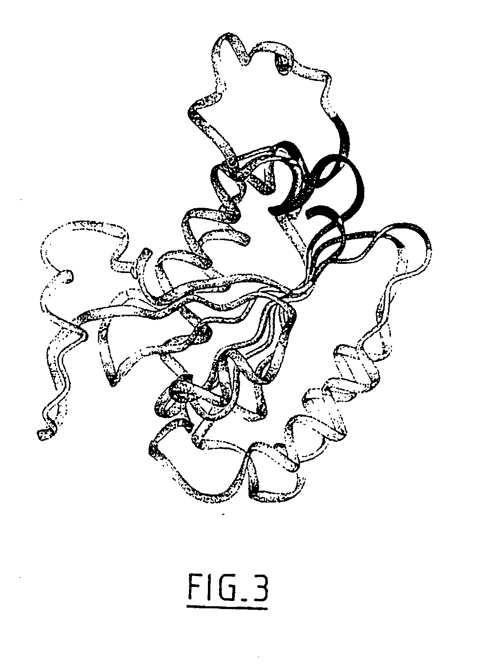

De préférence, ce composé sera un fragment d'un composé ABC transporteur de glutathion, notamment un fragment de la protéine CFTR comprenant le domaine NBF1 et plus particulièrement, un fragment comprenant le site potentiel de fixation du glutathion identifié dans les exemples qui suivent par référence à la figure 3. Il s'agit de préférence d'un fragment qui contient les résidus I448-Q452. S478-K481, M498-I506, A566-L568, A596-T599 localisés dans des boucles situées aux extrémités N terminales des feuillets 1 à 4 du domaine NBF1. L'administration conjointe d'un précurseur de glutathion est également avantageuse.

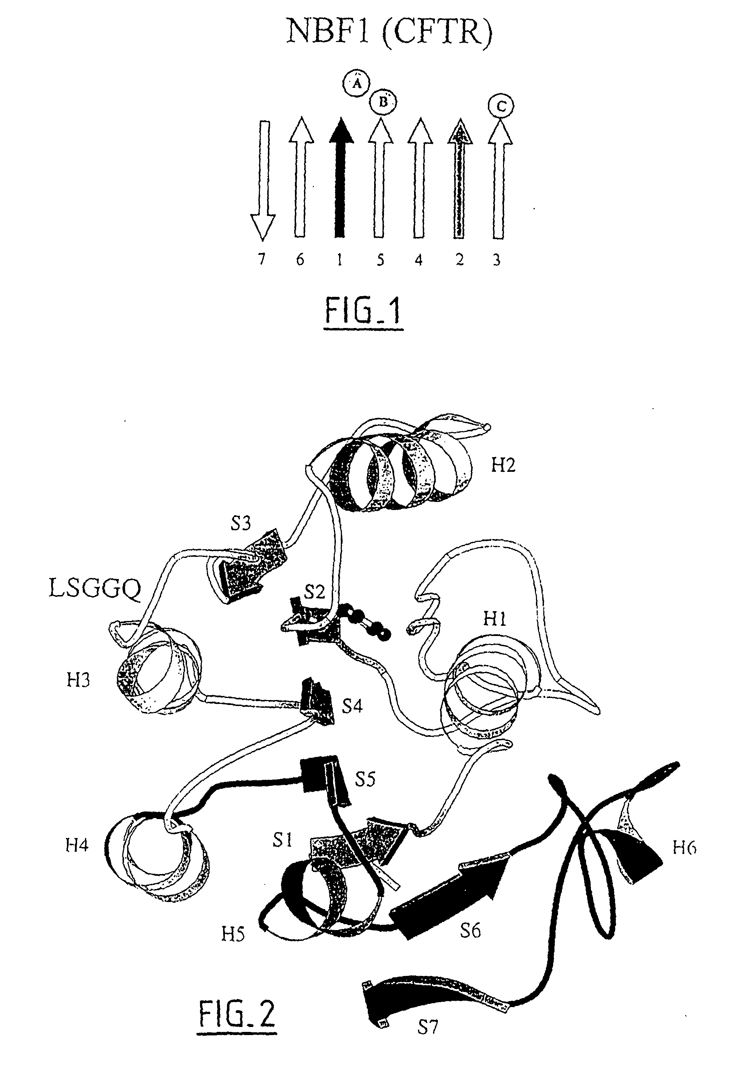

D'autres caractéristiques et avantages de l'invention apparaîtront au cours de la description détaillée suivante d'exemples de réalisation de l'invention, illustré par les figures 1 à 4 qui représentent la structure du domaine NBF1 de la protéine CFTR et la localisation du site potentiel de fixation du glutathion dans la protéine CFTR.

La figure 1 représente la topologie du feuillet central d'un modèle de NBF1. Il comporte un feuillet à six brins parallèles dont l'appariement est donné. Un septième brin est apparié en anti-parallèle (les brins 1 à 6 sont respectivement : L453-G458, N505-S511, N538-G542, D567-D572, R600-V603. Le brin 7 comprend les résidus K643-D648). Les positions des séquences consensus Walker A et B et la signature ABC sont indiquées.

La figure 2 représente schématiquement un modèle du domaine NBF1 de la protéine CFTR. Les feuillets 1 à 7 sont notés S1 à S7. Les hélices 1 à 6 sont indiquées H1 à H6. La position de la signature ABC (séquence LSGGQ) est indiquée. La position de la phénylalanine 508 est indiquée par une représentation CPK, sur le brin S2.

La figure 3 représente un modèle du domaine NBF1 de la protéine CFTR dans lequel les résidus potentiellement impliqués dans la fixation du glutathion sont représentés en gris foncé.

**EXEMPLE 1 : Etude de la structure de la protéine CFTR**

Méthode expérimentale

1. Construction du plasmide

On introduit le gène codant pour le domaine NBF1 de la protéine CFTR (séquence R450-1586) dans E.coli. Pour ce faire, le cADN de CFTR complet a été fourni par Transgène (Strasbourg, France). Le fragment d'ADN codant pour NBF1 a été amplifié par PCR. Pour la préamplification, les oligonucléotides 5'-GC AGA TCT AGA GGA CAG TTG TT-3' (SEQ ID n° 1) et 3'-TA CAA AAT TGT CTT TTT CTT ATT CTT AAG CG-5' (SEQ IN n° 2) ont été utilisés. Le produit d'amplification a été introduit par les sites de restriction Bam HI et Eco RI dans le plasmide pGEX-KT (Hakes et Dixon, Anal. Biochem., 202, 293-298, 1992). Dans ce plasmide, NBF1 est produit sous forme d'une protéine de fusion avec la Glutathion-S-Transférase (GST). Une séquence de liaison (linker) flexible polyglycine et un site de coupure à la thrombine séparent la GST et NBF1 dans la protéine de fusion. Le gène codant pour cette protéine de fusion a été séquencé et correspond bien au gène attendu. Le plasmide final à cette construction a été introduit dans une souche RecA-E.coli JM101TR.

2. Expression et purification de la protéine de fusion GST-NBF1 :

Les bactéries transformées ont été cultivées à 28°C dans un milieu de culture LB (Tryptone 15g, extrait de levure 5g, NaCL 8g, base Tris 0.52g, Ampiciline 100 mg par litre à pH 7.25). L'expression de la protéine de fusion a été induite par l'IPTG 0.1 mM, d'une DO de 0.8 à 2.5 à 600 nm (fréquence d'absorption des bactéries). Les bactéries ont été centrifugées à 4000 g pendant 25 minutes puis rincées deux fois avec du PBS à froid, avant sonication. Une resolubilisation a été obtenue avec une solution de PBS/0.1 mM et PMSF/Triton X100 1%. Les bactéries ont été soniquées sur un bain de glace pendant 5 minutes à 40W à l'aide d'une petite sonde.

La suspension a été centrifugée à 17000tr/min pendant 40 minutes à 4°C sur un rotor JA20 (Beckman). Le surnageant a été incubé avec 5ml d'une matrice de glutathion (G4B Pharmacia) à température ambiante pendant 30 minutes. La colonne a été remplie. L'élution de la protéine de fusion a été obtenue avec une solution 10 mM de glutathion et 50 mM de Tris à pH = 8, à une vitesse de passage de 0.05 ml/min. Les fractions absorbant à 280 nm ont été rassemblées et appliquées par portions de 500 μl sur une colonne échangeuse d'ions (MonoQ, Pharmacia). L'élution a été observée pour une solution de 150 mM de NaCl, et une protéine de 42 kD a été identifiée sur gel SDS-PAGE, puis confirmée par spectroscopie de masse. Les rendements de production en protéine de fusion soluble sont de 5mg/l de culture.

3. Coupure de la protéine de fusion GST-NBF1 :

Après purification, l'échantillon de protéine de fusion a été dialysé contre un tampon (150 mM NaCl, CaCl2 2.5 mM, Tris/HCl 50 mM) à pH = 8. La thrombine humaine (T7009, Sigma) a été additionnée jusqu'à une concentration finale de 5u par mg de protéine de fusion. Le mélange a été incubé 30 minutes à température ambiante. Les produits de la réaction de coupure ont été caractérisés sur gel SDS-PAGE, et une protéine de poids moléculaire 18kD (correspondant à celui attendu pour NBF1) a été observée.

Résultats :

La protéine de fusion GST-NBF1 est effectivement reconnue par un anti-corps anti-NBF1 (MATG 1061, distribué par Transgène). L'affinité de cette protéine recombinante pour l'ATP a été vérifiée. Pour ce faire, la reconnaissance d'un dérivé fluorescent de l'ATP (le TNP-ATP) a été mise en évidence. La protéine recombinante a donc été produite sous une forme fonctionnelle. Cependant, après coupure à la thrombine, il n'est pas possible de séparer la GST et le domaine NBF1 sur colonne d'affinité greffée au glutathion (GSH) selon un mode expérimental analogue à celui décrit ci-dessus. Bien que parfaitement séparées sur gel d'électrophorèse, les protéines NBF1 et GST co-éluent sur colonne de GSH immobilisé. Cette observation suppose que (comme prévu pour la GST) NBF1 est retenu sur la colonne de GSH, ce qui concorde avec l'existence d'un site de fixation du glutathion dans NBF1.

**EXEMPLE 2 : Mise en évidence dans le domaine NBF1 de la présence d'un site potentiel de fixation du glutathion**

Un modèle de structure pour le domaine NBF1 de CFTR (Annereau et al., C.R. Acad. Sci. Paris, Sciences de la Vie/Life Sciences, 320, 113-121, 1997 et Annereau et al., FEBS Letters, 407, 303-308, 1997) a été construit par homologie avec la structure connue des domaines de fixation des nucléotides de l'ATPase-F1 de boeuf (Abrahams J. P., Leslie A.G.W., Lutter R., Walker J.F., 1994 Nature, 370, 621-628). Ce domaine comprend un coeur hydrophobe constitué d'un feuillet β à 6 brins parallèles, dont l'appariement par rapport à l'ordre dans la séquence primaire est donné figure 1 (les brins 1 à 6 sont respectivement : L453-G458, N505-S511, N538-G542, D567-D572, R600-V603). Ces feuillets alternent avec 6 hélices α (K464-E474, Y517-D529, Q552-Y563, L581-V591, S605-K611, S631-L636). Les hélices α s'organisent de part et d'autre du plan moyen du feuillet β central, comme montré figure 2 (FEBS Letters, vol 407, pp 303-308, 1997). Des boucles situées en surface du domaine relient les hélices aux feuillets.

L'existence potentielle d'un site de fixation du glutathion dans NBF1 de CFTR pratiquement superposable au site de fixation du glutathion dans la GST est mise en évidence par comparaison de ce modèle avec la structure connue de la GST (Glutathion-S-Transférase, protéine qui présente un site de fixation du glutathion et qui catalyse les réactions d'attaques nucléophiles du glutathion). La figure 3 montre les résidus de NBF1 potentiellement impliqués dans la fixation du glutathion. Ils se situent dans une région formant une crevasse aux extrémités de brins impliqués dans un feuillet β Ces résidus sont situés plus précisément dans des boucles, aux extrémités N terminales des feuillets 1 à 4 de la structure de NBF1. Ces boucles contiennent les résidus 1448-Q452, S478-K481, M498-I506, A566-L568, A596-T599.

**EXEMPLE 3 : Traitement de la mucoviscidose par une association d'épirubicine et de cyclophosphamide**

Le traitement anti-tumoral suivant a été administré :

.    épirubicine (Farmorubicine®), 110 mg pendant deux jours (J1 et J2) ;
.    ifosfamide (Holoxan®), 3,3 g pendant cinq jours (J1 à J5)
.    Filgrastime (Neupogen®), 300 μg par jour pendant huit jours (J8 à J15) ;

sous couvert de :

.    granisétron (Kytril®), pour la prévention des nausées ;
.    Méthylprednisolone (Solumédrol®, corticoïde) ;
.    Mesna (Mucofluid ®) ;
.    puis, trois mois plus tard, le malade a reçu à nouveau six cycles épirubicine et ifosfamide.

Le malade traité présentait un génotype ΔF 508 une mutation correspondant à une délétion d'une phénylalanine en position 508 dans l'exon 12 de CFTR sur un allèle et, une autre mutation G673X, située dans l'exon 13 sur l'autre allèle. Ce malade présentait, depuis sa naissance en 1968, tous les signes cliniques associés à la maladie : tests de la sueur positifs, sinusites répétées, bronchites répétées, polypes de la fosse nasale etc... En 1989 il a présenté une infection à Pseudomonas aeruginosa, classique dans la mucoviscidose et habituellement pratiquement définitive chez ces malades. En outre, en avril 1993, un fibrosarcome de la cuisse gauche a été diagnostiqué. Le malade a subi un traitement chirurgical et radiothérapique puis une chimiothérapie de juillet à octobre 1993, suivis d'un nouveau traitement en janvier 1994. Concernant le tableau clinique de la mucoviscidose, à la suite du traitement chimiothérapique, on a observé les améliorations suivantes :

-    l'état respiratoire du malade s'est amélioré considérablement ;
-    son infection par Pseudomonas aeruginosa a disparu ;
-    ses paramètres respiratoires atteignent environ 75 % des valeurs théoriques ;
-    il a recouvré un état respiratoire à peu près équivalent à celui qu'il présentait au début de son adolescence ;
-    il se déclare lui-même guéri de la mucoviscidose.

Un test de la sueur effectué chez lui au début de l'année 1997 s'est révélé toujours très positif, ce qui signifie que la fonction de canal à ion chlorure n'a pas été rétablie chez ce patient. Cette constatation, liée au fait que le malade se déclare lui-même guéri de la mucoviscidose permet de conclure qu'une autre fonction essentielle a été rétablie par le traitement chimiothérapique. Grâce aux travaux menés par les inventeurs, on peut maintenant supposer qu'il s'agit d'une fonction de transport au moyen du glutathion.

EP 0 984 784 B1

**EXEMPLE 4 : Mise en évidence de la surexpression des protéines MDR et MRP chez le patient après traitement chimiothérapique conformément à l'exemple 3**

L'ARNm des protéines CETR, MDR et MRP a été dosé par RT-PCR sur des cellules éphithéliales collectées et analysées chez le patient de l'exemple 3 (patient A). Le même dosage a été effectué sur un patient atteint de mucoviscidose qui n'a jamais été exposé à des produits anti-cancéreux (patient B).

Chez le patient B, les ARNm de CFTR, MDR et MRP étaient indétectables mais ils ont été identifiés sans ambiguité chez le patient A.

Ces résultats renforcent l'hypothèse à la base de l'invention selon laquelle l'amélioration de l'état clinique du malade serait due à la substitution de CFTR par MDR et/ou MRP. En effet, même surexprimée, la protéine CFTR mutée n'est a priori pas fonctionnelle.

**EXEMPLE 5 : Traitement de la mucoviscidose par chimiothérapie prescrite pour un lymphome.**

Le malade, né en 1969, est atteint d'une mucoviscidose, diagnostiquée à l'âge de deux mois par un test de la sueur positif. Son tableau clinique est typique de la mucoviscidose (insuffisance pancréatique externe, polypose des sinus, colonisation chronique à pyocyanique). Il suit un traitement de fond standart pour sa mucoviscidose : kinésithérapie respiratoire, plusieurs cures par an d'antibiothérapie, vitamines. En août 1992, il présente un lymphome de stade IV. Il subit quatre cures associant adriamycine, cyclophosphamide (Endoxan Asta®) cytarabine (Aracytine®), vincristine (Oncovin®), methylprednisolone (Dépa-medrol®), Bléomycine, Méthotrexate en octobre 1992 et novembre 1992. En janvier 1993, il subit un nouveau traitement associant méthotrexate, cytarabine et methylprednisolone. Tout traitement chimiothérapique est arrêté en avril 1993. Sur le plan pulmonaire, le malade est jugé transformé de mars 1993 à décembre 1993, par lui-même et par les médecins qui le suivent ; plus de toux, plus de kinésithérapie respiratoire, il fait des efforts physiques sans problèmes, et il présente plusieurs examens pulmonaires normaux. En l'absence de traitement chimiothérapique d'entretien, certains symptômes ont repris depuis fin 1994, tels que majoration de la toux. Cependant, on a noté une amélioration notable de l'état du patient, sur une période d'un an suivant le traitement d'attaque pour le lymphome.

Le malade traité était homozygote pour une mutation dans l'exon 20 au sein du domaine NBF2.

**Revendications**

1. Utilisation, pour la préparation d'un médicament destiné à la prévention et/ou au traitement de la mucoviscidose, d'au moins un produit dont l'administration à un patient atteint de mucoviscidose, conduit chez ledit patient à l'expression ou à la surexpression d'au moins un composé transporteur ABC.

2. Utilisation selon la revendication 1 **caractérisée en ce que** ledit produit conduit chez ledit patient à l'expression ou à la surexpression de la protéine CETR mutée du patient.

3. Utilisation selon la revendication 1, **caractérisée en ce que** ledit composé exprimé ou surexprimé est la protéine MDR.

4. Utilisation selon la revendication 1, **caractérisée en ce que** ledit composé exprimé ou surexprimé est un composé ABC transporteur de glutathion.

5. Utilisation selon la revendication 4, **caractérisée en ce que** ledit composé exprimé ou surexprimé est la protéine MRP.

6. Utilisation selon l'une des revendications 1 à 5, **caractérisée en ce que** ledit composé exprimé ou surexprimé est la protéine MRP et/ou la protéine MDR et/ou la protéine CFTR.

7. Utilisation selon l'une des revendications 1 à 6, **caractérisée en ce que** ledit produit est un produit anti-cancéreux.

8. Utilisation selon la revendication 7, **caractérisée en ce que** ledit produit anti-cancéreux est un agent alkylant.

9. Utilisation selon la revendication 8, **caractérisée en ce que** ledit produit est choisi parmi les moutardes azotées, les nitroso-urées, les dérivés de platine, les dérivés de l'éthylène-émine, les diméthane sulfonoxyalcanes, les dérivés de la pipérazine et les dérivés de la méthylhydrazine.

10. Utilisation selon la revendication 9, **caractérisée en ce que** ledit produit est une moutarde azotée, de préférence le cyclophosphamide.

11. Utilisation selon la revendication 7, **caractérisée en ce que** ledit produit anti-cancéreux est un agent intercalant.

12. Utilisation selon la revendication 11, **caractérisée en ce que** ledit produit est choisi parmi les anthracyclines, les anthracérédiones, les anthracènes, les dérivés de l'acridine, les ellipticines, les actinomycines.

13. Utilisation selon la revendication 12, **caractérisée en ce que** ledit produit est une anthracycline.

14. Utilisation selon la revendication 13, **caractérisée en ce que** ledit produit est choisi parmi l'aclarubicine, la doxorubicine, la daunorubicine, l'épirubicine, l'idarubicine, la zorubicine, la pirabucine.

15. Utilisation selon la revendication 14, **caractérisée en ce que** ledit produit est l'épirubicine.

16. Utilisation selon la revendication 7, **caractérisée en ce que** ledit produit anti-cancéreux est un antimétabolite.

17. Utilisation selon la revendication 7, **caractérisée en ce que** ledit composé anti-cancéreux est un poison du fuseau.

18. Utilisation selon la revendication 17, **caractérisée en ce que** ledit poison du fuseau est la colchicine ou ses dérivés.

19. Utilisation selon la revendication 17, **caractérisée en ce que** ledit produit est choisi parmi les épipodophyllotoxines et les vinca-alcaloïdes.

20. Utilisation selon la revendication 19, **caractérisée en ce que** ledit produit est choisi parmi la vindésine, la vinorelbine, la vincristine et la vinblastine.

21. Utilisation selon l'une des revendications 1 à 6, **caractérisée en ce que** ledit produit est un analogue d'un agent anti-cancéreux.

22. Utilisation selon l'une des revendications 1 à 6, **caractérisée en ce que** ledit produit est un macrolide.

23. Utilisation selon la revendication 22, **caractérisée en ce que** ledit produit est l'azithromycine.

24. Utilisation selon la revendication 22, **caractérisée en ce que** ledit produit est l'erythromycine.

25. Utilisation, selon l'une des revendications 7 à 24, **caractérisée en ce que** ledit médicament contient au moins un agent anti-cancéreux et au moins un macrolide comme produit de combinaison pour une utilisation simultanée, séparée ou étalée dans le temps.

26. Utilisation selon la revendication 25, **caractérisée en ce que** ledit produit de combinaison contient un agent alkylant et un agent intercalant.

27. Utilisation selon la revendication 26, **caractérisée en ce que** l'agent alkylant est l'ifosfamide et l'agent intercalant est l'épirubicine

**28.** Utilisation selon l'une des revendications 1 à 27, **caractérisée en ce que** ledit produit est en combinaison avec un précurseur de glutathion, comme produit de combinaison pour une utilisation simultanée, séparée ou étalée dans le temps.

**29.** Utilisation selon la revendication 28, **caractérisée en ce que** ledit précurseur de glutathion est choisi parmi la N-acétyle cystéine ou la N-acétyle lysine.

**Claims**

**1.** Use, for preparing a medicament which is intended for preventing and/or treating cystic fibrosis, of at least one product whose administration to a patient suffering from cystic fibrosis leads, in the said patient, to the expression or overexpression of at least one ABC transporter compound.

**2.** Use according to Claim 1, **characterized in that** the said product leads, in the said patient, to the expression or overexpression of the patient's mutated CFTR protein.

**3.** Use according to Claim 1, **characterized in that** the said expressed or overexpressed compound is the MDR protein.

**4.** Use according to Claim 1, **characterized in that** the said expressed or overexpressed compound is an ABC glutathione transporter compound.

**5.** Use according to Claim 4, **characterized in that** the said expressed or overexpressed compound is the MRP protein.

**6.** Use according to one of Claims 1 to 5, **characterized in that** the said expressed or overexpressed compound is the MRP protein and/or the MDR protein and/or the CFTR protein.

**7.** Use according to one of Claims 1 to 6, **characterized in that** the said product is an anticancer product.

**8.** Use according to Claim 7, **characterized in that** the said anticancer product is an alkylating agent.

**9.** Use according to Claim 8, **characterized in that** the said product is selected from nitrogen mustards, nitrosoureas, platinum derivatives, ethyleneimine derivatives, dimethane sulphonoxyalkanes, piperazine derivatives and methylhydrazine derivatives.

**10.** Use according to Claim 9, **characterized in that** the said product is a nitrogen mustard, preferably cyclophosphamide.

**11.** Use according to Claim 7, **characterized in that** the said anticancer product is an intercalating agent.

**12.** Use according to Claim 11, **characterized in that** the said product is selected from anthracyclines, anthracerediones, anthracenes, acridine derivatives, ellipticines and actinomycins.

**13.** Use according to Claim 12, **characterized in that** the said product is an anthracycline.

**14.** Use according to Claim 13, **characterized in that** the said product is selected from aclarubicin, doxorubicin, daunorubicin, epirubicin, idarubicin, zorubicin and pirabucin.

**15.** Use according to Claim 14, **characterized in that** the said product is epirubicin.

**16.** Use according to Claim 7, **characterized in that** the said anticancer product is an antimetabolite.

**17.** Use according to Claim 7, **characterized in that** the said anticancer compound is a spindle poison.

**18.** Use according to Claim 17, **characterized in that** the said spindle poison is colchicine or its derivatives.

**19.** Use according to Claim 17, **characterized in that** the said product is selected from epipodophyllotoxins and vinca alkaloids.

**20.** Use according to Claim 19, **characterized in that** the said product is selected from vindesine, vinorelbine, vincristine and vinblastine.

**21.** Use according to one of Claims 1 to 6, **characterized in that** the said product is an analogue of an anticancer agent.

**22.** Use according to one of Claims 1 to 6, **characterized in that** the said product is a macrolide.

**23.** Use according to Claim 22, **characterized in that** the said product is azithromycin.

**24.** Use according to Claim 22, **characterized in that** the said product is erythromycin.

**25.** Use according to one of Claims 7 to 24, **characterized in that** the said medicament contains at least one anticancer agent and at least one macrolide as a combination product for simultaneous or separate use or use which is staggered over time.

**26.** Use according to Claim 25, **characterized in that** the said combination product contains an alkylating agent and an intercalating agent.

**27.** Use according to Claim 26, **characterized in that** the alkylating agent is ifosfamide and the intercalating agent is epirubicin.

**28.** Use according to one of Claims 1 to 27, **characterized in that** the said product is combined with a glutathione precursor, as a combination product for simultaneous or separate use or use which is staggered over time.

**29.** Use according to Claim 28, **characterized in that** the said glutathione precursor is selected from N-acetylcysteine or N-acetyllysine.


**Patentansprüche**

**1.** Verwendung für die Herstellung eines Medikaments, das zur Verhütung und/oder zur Behandlung von Mukoviszidose bestimmt ist, mindestens eines Mittels, dessen Verabreichung bei einem Patienten, der an Mukoviszidose leidet, bei dem Patienten zur Expression oder Überexpression mindestens einer ABC-Transportverbindung führt.

**2.** Verwendung nach Anspruch 1, **dadurch gekennzeichnet, dass** das Mittel bei dem Patienten zur Expression oder Überexpression des mutierten CFTR-Proteins des Patienten führt.

**3.** Verwendung nach Anspruch 1, **dadurch gekennzeichnet, dass** die exprimierte oder überexprimierte Verbindung das MDR-Protein ist.

**4.** Verwendung nach Anspruch 1, **dadurch gekennzeichnet, dass** die exprimierte oder überexprimierte Verbindung eine ABC-Transportverbindung für Glutathion ist.

**5.** Verwendung nach Anspruch 4, **dadurch gekennzeichnet, dass** die exprimierte oder überexprimierte Verbindung das MRP-Protein ist.

**6.** Verwendung nach einem der Ansprüche 1 bis 5, **dadurch gekennzeichnet, dass** die exprimierte oder überexprimierte Verbindung das MRP-Protein und/oder das MDR-Protein und/oder das CFTR-Protein ist.

**7.** Verwendung nach einem der Ansprüche 1 bis 6, **dadurch gekennzeichnet, dass** das Mittel ein Antikrebs-Mittel ist.

**8.** Verwendung nach Anspruch 7, **dadurch gekennzeichnet, dass** das Antikrebs-Mittel ein Alkylierungsmittel ist.

**9.** Verwendung nach Anspruch 8, **dadurch gekennzeichnet, dass** das Mittel ausgewählt ist aus Stickstofflost und dessen Derivaten, Nitrosoharnstoffen, Platin-Derivaten, Derivaten von Ethylenimin, Dimethansulfonoxyalkanen, Derivaten von Piperazin und Derivaten von Methylhydrazin.

**10.** Verwendung nach Anspruch 9, **dadurch gekennzeichnet, dass** das Mittel ein Stickstofflost oder ein Derivat davon ist, vorzugsweise Cyclophosphamid.

**11.** Verwendung nach Anspruch 7, **dadurch gekennzeichnet, dass** das Antikrebs-Mittel ein Interkalationsmittel ist.

**12.** Verwendung nach Anspruch 11, **dadurch gekennzeichnet, dass** das Mittel ausgewählt ist aus Anthracyclinen, Anthracaredionen, Anthracenen, Acridin-Derivaten, Ellipticinen, Actinomycinen.

**13.** Verwendung nach Anspruch 12, **dadurch gekennzeichnet, dass** das Mittel ein Anthracyclin ist.

**14.** Verwendung nach Anspruch 13, **dadurch gekennzeichnet, dass** das Mittel ausgewählt ist aus Aclarubicin, Doxorubicin, Daunorubicin, Epirubicin, Idarubicin, Zorubicin, Pirabucin.

**15.** Verwendung nach Anspruch 14, **dadurch gekennzeichnet, dass** das Mittel Epirubicin ist.

**16.** Verwendung nach Anspruch 7, **dadurch gekennzeichnet, dass** das Antikrebs-Mittel ein Antimetabolit ist.

**17.** Verwendung nach Anspruch 7, **dadurch gekennzeichnet, dass** die Antikrebs-Verbindung ein Spindelgift ist.

**18.** Verwendung nach Anspruch 17, **dadurch gekennzeichnet, dass** das Spindelgift Colchicin oder seine Derivate ist.

**19.** Verwendung nach Anspruch 17, **dadurch gekennzeichnet, dass** das Mittel ausgewählt ist aus Epipodophyllotoxinen und Vinca-Alcaloiden.

**20.** Verwendung nach Anspruch 19, **dadurch gekennzeichnet, dass** das Mittel ausgewählt ist aus Vindesin, Vinorelbin, Vincristin und Vinblastin.

**21.** Verwendung nach einem der Ansprüche 1 bis 6, **dadurch gekennzeichnet, dass** das Mittel ein Analogon eines Antikrebs-Mittels ist.

**22.** Verwendung nach einem der Ansprüche 1 bis 6, **dadurch gekennzeichnet, dass** das Mittel ein Makrolid ist.

**23.** Verwendung nach Anspruch 22, **dadurch gekennzeichnet, dass** das Mittel Azithromycin ist.

**24.** Verwendung nach Anspruch 22, **dadurch gekennzeichnet, dass** das Mittel Erythromycin ist.

**25.** Verwendung nach einem der Ansprüche 7 bis 24, **dadurch gekennzeichnet, dass** das Medikament mindestens ein Antikrebs-Mittel und mindestens ein Makrolid als Kombinationsprodukt für eine gleichzeitige, getrennte oder zeitlich gestaffelte Verwendung enthält.

**26.** Verwendung nach Anspruch 25, **dadurch gekennzeichnet, dass** das Kombinationsprodukt ein Alkylierungsmittel und ein Interkalationsmittel enthält.

**27.** Verwendung nach Anspruch 26, **dadurch gekennzeichnet, dass** das Alkylierungsmittel Ifosfamid ist und das Interkalationsmittel Epirubicin ist.

**28.** Verwendung nach einem der Ansprüche 1 bis 27, **dadurch gekennzeichnet, dass** das Mittel in Kombination mit einer Glutathion-Vorstufe als Kombinationsprodukt für eine gleichzeitige, getrennte oder zeitlich gestaffelte Verwendung vorliegt.

**29.** Verwendung nach Anspruch 28, **dadurch gekennzeichnet, dass** die Glutathion-Vorstufe ausgewählt ist aus N-Acetylcystein oder N-Acetyllysin.

NBF1 (CFTR)

FIG_1

FIG_2

FIG.3